# NEUE EUROPÄISCHE PATENTSCHRIFT

(11) **EP 1 524 948 B2**
(45) Veröffentlichungstag und Bekanntmachung des Hinweises auf die Entscheidung über den Einspruch: **02.05.2018**
(45) Hinweis auf die Patenterteilung: 07.05.2014
(21) Anmeldenummer: 03766132.9
(22) Anmeldetag: 08.07.2003
(51) Int. Cl.: A61C 8/00

(54) **DENTALIMPLANTAT MIT VERANKERUNGSKOPF UND SCHRAUBKÖRPER**
DENTAL IMPLANT COMPRISING AN ANCHORING HEAD AND A SCREW ELEMENT
IMPLANT DENTAIRE A TETE D'ANCRAGE ET CORPS A VISSER

(30) Priorität: 26.07.2002 DE 10234113; 21.08.2002 DE 10238091
(43) Veröffentlichungstag der Anmeldung: 27.04.2005
(73) Patentinhaber: Bredent Dentalgeräte und Materialien Fach- und Organisationsberatung Peter Brehm, 89250 Senden (DE)
(72) Erfinder: LANG, Manfred, 90518 Altdorf (DE); LERMER, Jörg, 90419 Nürnberg (DE); KOCH, Andreas, 8804 Au (CH)
(74) Vertreter: Dziewior, Joachim
(86) Internationale Anmeldenummer: PCT/EP2003/007288
(87) Internationale Veröffentlichungsnummer: WO 2004/012622

(56) Entgegenhaltungen:
- EP-A- 0 263 809
- EP-A- 1 243 229
- WO-A-99/17676
- WO-A-03/015654
- DE-U- 20 013 654
- DE-U- 20 112 507
- DE-U1- 20 113 254
- US-A- 5 593 410
- US-A- 5 816 812
- US-A- 5 871 356
- US-A- 6 135 773
- US-A- 6 135 773
- US-A1- 2002 039 717

## Beschreibung

Die Erfindung betrifft ein Dentalimplantat mit einem Verankerungskopf für ein Aufbauteil und mit einem Schraubkörper umfassend einen Gewindekern und ein selbstschneidendes Außengewinde.

Gewindekonfigurationen für den Schraubkörper von Dentalimplantaten sind aus einer Vielzahl von Druckschriften zum Stand der Technik bekannt wie z.B. US 5816812 und WO99/17676. So offenbart die DE 201 13 254 U1 ein Zylinderschraubimplantat, bei dem ausgehend von einem zylindrischen Verankerungskopf der Gewindekern von crestal nach apikal im Durchmesser konisch abnimmt, während das selbstschneidende Außengewinde über seine Länge einen konstanten Außendurchmesser aufweist. Aufgrund der damit von apikal nach crestal immer tiefer werdenden Gewindegänge können sich am apikalen Ende des implantierten Schraubkörpers zwischen der Innenwand der zylindrischen Finalbohrung im Kieferknochen und dem Gewindekern Hohlräume bilden, was aus medizinischen Gründen nachteilig ist.

Bei der aus der DE 37 35 378 C2 bekannten Gestaltung des Schraubkörpers sind im Außendurchmesser über die größte Länge konstante Gewindekern und Außengewinde vorgesehen. Lediglich zum apikalen Ende des Schraubkörpers hin weisen Gewindekern und Außengewinde einen leicht konischen Verlauf auf. Durch die große Gewindelänge mit konstantem Außendurchmesser werden beim Einschrauben dieses vorbekannten Dentalimplantates mit zunehmender Einschraubtiefe relativ große Kräfte auf den Kieferknochen wirksam, wodurch eine relativ starke Traumatisierung des beaufschlagten Knochengewebes hervorgerufen werden kann.

Die DE 36 42 901 A1 offenbart ein Sondergewinde für Dentalimplantate, bei der ein relativ stark konisch zulaufender Gewindekern mit einem Außengewinde kombiniert ist, das bis auf einen kurzen konischen Zulauf am apikalen Ende mit einem konstanten Außendurchmesser versehen ist. Auch hier ergibt sich wieder das Problem der großen Gewindetiefe im Bereich vor dem apikalen Ende.

Die EP 0 282 789 B1 schließlich bezieht sich auf ein selbstschneidendes, einschraubbares Knochenimplantat für zahnärztliche Zwecke, das zeichnerisch eine Gewindekonfiguration offenbart, die dem erstgenannten Gebrauchsmuster entspricht. Insoweit ist also ein konisch zulaufender Gewindekern mit einem Außengewinde kombiniert, dessen Außendurchmesser konstant und dort insbesondere gleich dem Außendurchmesser der zylindrischen Pfostenverankerung ist.

Als weitere Alternative für den Schraubkörper ist in dieser Druckschrift ein Gewindeverlauf beansprucht, wonach der Außendurchmesser des Außengewindes, der am Ansatz gleich dem Durchmesser des zylindrischen Verankerungskopfes ist, sich zum freien Ende des Kerns hin stetig vermindert, wobei ein konischer Gewindekern vorgesehen sein soll. Mit anderen Worten wird also ein konisch zulaufendes Außengewinde mit einem ebenfalls konischen Kern kombiniert. Durch die stetige Durchmesserverringerung, also das Fehlen jeglicher zylindrischer Abschnitte in Gewindekern und/oder Außengewinde kann die Stabilität und innige Verbindung zwischen Implantat und umgebender Knochengewebe beeinträchtigt sein.

Wie aus dem druckschriftlichen Stand der Technik zwar nicht explizit hervorgeht, jedoch aus der aktuellen Praxis der Anwendung von Dentalimplantaten bekannt ist, werden die im Kieferknochen anzulegenden Bohrungen für das Dentalimplantat in aller Regel mit einem solchen Durchmesser ausgeführt, dass allenfalls die äußersten Spitzen der Gewindestege in das Knochengewebe eindringen. Die Gewindegänge dienen dann als Vertiefungen, um zur Stabilisierung des Implantatssitzes Knochengewebe dort ineinwachsen zu lassen. Nachvollziehbar ist insbesondere die Primärstabilität eines solchen Dentalimplantates verbesserungsbedürftig.

Ausgehend von der geschilderten Problematik beim Stand der Technik liegt der Erfindung die Aufgabe zugrunde, ein Dentalimplantat bezüglich seines Schraubkörpers so zu verbessern, dass eine möglichst stabile Verankerung bei gleichzeitig möglichst geringer Beanspruchung des das Implantat umgebenden Knochenbereiches erzielt wird.

Diese Aufgabe wird durch die im Kennzeichnungsteil des Anspruches 1 angegebene, spezielle Gewindekonfiguration erzielt.

Es werden also zylindrische und konische Gewindeverläufe sowohl beim Gewindekern als auch beim Außengewinde verwendet, deren Kombination eine jeweilige Funktionsoptimierung der einzelnen Gewindeabschnitte erzielt. So wird im apikal gelegenen Spitzenabschnitt durch eine kontinuierliche Vergrößerung des Schneidquerschnitts und des Kerndurchmessers ein sanfter Anschnitt des im Knochen anzulegenden Gegengewindes erzielt. Der Mittenabschnitt mit einem praktisch zylindrischen Gewindeteil ohne konische oder irgendwie stetig abnehmende Verläufe von Gewindekern bzw. Außengewinde ergibt eine gute Stabilität, dabei jedoch gleichzeitig ausgeglichene Belastung des umgebenden Materials durch die uniforme Flächenpressung der Gewindegänge. Schließlich sorgt der Crestalabschnitt für einen sanften Übergang vom Schraubkörper zum Verankerungskopf, sodass dort scharfe Kanten und entsprechende Spannungskonzentrationen sowie Kerbwirkung auf das Knochengewebe vermieden werden.

Dies ist besonders dann der Fall, wenn - wie in einer bevorzugten Ausführungsform vorgesehen ist - der Crestalabschnitt mit seinem konischen Gewindekern stufenlos in den Verankerungskopf übergeht. Bei der Implantatinsertion erlaubt die beanspruchte Gewindekonfiguration eine ideale Knochenanlagerung, wodurch eine optimale Primärstabilität bei allen Knochendichten erreicht wird. Diese Primärstabilität kann dabei durch eine subtraktive Oberflächenvergrößerung erhöht werden. Im Gegensatz zu additiven Oberflächenaufrauhungen, wie das Aufbringen von Partikelbeschichtungen, verhindert dies die gefährliche Abscherung von solchen Teilen - beispielsweise Titanteilchen - insbesondere bei selbstschneidenden Gewinden während der Insertion.

Erfindungsgemäß ist das Außengewinde des Schraubkörpers zweigängig angelegt, was ein schonenderes Einbringen des Implantates mit weniger Knochenabrieb erlaubt. Daraus resultiert wiederum eine verkürzte Einheilzeit des Implantates.

Erfindungsgemäß liegt das Verhältnis der Axiallängen von Crestal- bzw. Spitzenabschnitt einerseits zum Mittenabschnitt andererseits zwischen 1:1 und 1:2. In Abhängigkeit vom jewelligen Durchmesser des Dentalimplantates werden so die eingangs erwähnten Verankerungs- und Einschraubeigenschaften erzielt. Demselben Zweck dient das bevorzugte Verhältnis der Gewindeaußendurchmesser in Crestal- und Mittenabschnitt zum apikalen Ende des Spitzenabschnittes von etwa 4:3.

Zur Verankerung eines jeweiligen Aufbauteils ist gemäß einer weiteren bevorzugten Ausführungsform der Erfindung am Verankerungskopf eine Innensechsrund-artige Verzahnung - eine sogenannte "Torx®-Verzahnung" vorgesehen -, der im Gegensatz zu sechskantförmigen Innenverzahnungen aufgrund seiner gerundeten, parallel zur Schraubenachse stehenden Kraftangriffsflächen wesentlich geringere Flächenpressungen aufweist, sodass Beschädigungen am Einbringwerkzeug und an den Implantataufbauten auszuschließen sind. Es wird somit eine höhere Sicherheit bei voller Kraftübertragung geboten. Darüber hinaus erreicht die Innensechsrund-Verzahnung bei gleicher Fertigungstoleranz gegenüber dem Innensechskant eine deutlich bessere Verdrehsicherheit. Ferner wird beim Innensechskant durch den Angriffswinkel von 60° auf die einzelnen Zähne ein großer Teil des Drehmoments in Radialkräfte umgewandelt, die gegen die Fläche wirken und als Schraubkraft verloren gehen. Insgesamt erzielt die Innensechsrund-Verzahnung also eine leichtgängige Eindrehbarkeit des Implantats mit anschließender hochstabiler Drehverankerung des in der Innenverzahnung sitzenden Aufbauteils.

Ferner wird ein Satz von Dentalimplantaten offenbart, bei dem die crestal weisenden Verankerungsflächen der Verankerungsköpfe mindestens zweier im Gewinde-Außendurchmesser unterschiedlicher Dentalimplantate einen gemeinsamen Normal-Außendurchmesser aufweise. Diesen Satz-Konfiguration ist dabei mit Vorteil auch bei solchen Dentalimplantaten zu verwirklichen, die nicht die im Kennzeichnungsteil des Anspruches 1 angegebene Gewindekonfiguration aufweisen.

Als vorteilhafte Satzgröße hat sich dabei ein Dreiersatz in Form eines Normalgewinde-, eines Schmalgewinde- und eines Breitgewinde-Implantats herausgestellt, wobei sich im Bereich des zylindrischen Verankerungskopfes alle drei Implantatdurchmesser auf einen gemeinsamen Normal-Außendurchmesser des Verankerungskopfes eichen. Dies hat den erheblichen Vorteil, dass ein und dasselbe Aufbauteil für drei unterschiedliche Implantatdurchmesser verwendet werden kann, ohne dabei Durchmesserstufen, scharfe Rück- oder Vorsprünge oder dergleichen zwischen Aufbauteil und Verankerungskopf in Kauf nehmen zu müssen. In der Implantatpraxis werden dementsprechend die Kosten der Lagerhaltung für Aufbauteile entsprechend der Satzanzahl gesenkt - bei einem Dreiersatz also auf ein Drittel. Insoweit wird die bei bekannten Implantatsystemen oft unüberschaubare Vielfalt an Aufbauteilen und Implantatzubehörteilen drastisch reduziert und auf das Wesentliche beschränkt.

Schließlich ist ein Finalbohrer zum Anlegen einer Finalbohrung für ein Dentalimplantat vorgesehen, der eine konische Schneidkontur mit dem Konusverlauf des Gewindekerns angepassten Konuswinkel und einen Außendurchmesser aufweist, der dem Außendurchmesser des Gewindekerns entspricht. Durch die konische Form wird dabei erreicht, dass das in die Finalbohrung eingesetzte Dentalimplantat vor dem eigentlichen Einschraubvorgang bereits gut in seiner Sollrichtung ausgerichtet ist und praktisch "wackelfrei" sitzt. Beim anschließenden Einschrauben schneiden sich die Gewindestege des Außengewindes zunehmend in das Knochengewebe ein, sodass die Gewindegänge initial gefüllt sind. Damit wird eine hohe Primärstabilität erreicht.

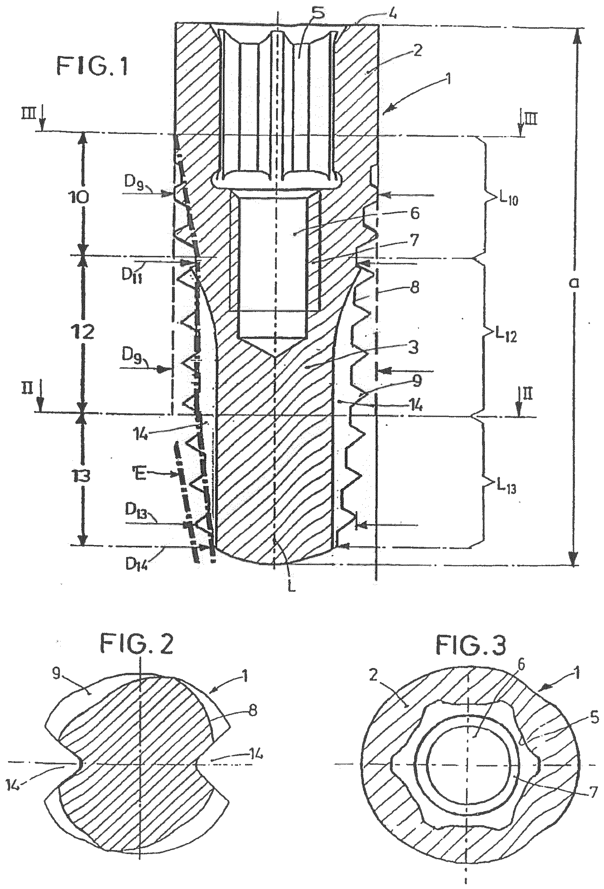

Weitere Merkmale, Vorteile und Einzelheiten der Erfindung ergeben sich aus der nachfolgenden Beschreibung, in der ein Ausführungsbeispiel anhand der beigefügten Zeichnungen näher erläutert wird. Es zeigen:
Fig. 1
   einen Längsaxialschnitt durch ein Dentalimplantat,
Fig. 2
   einen Querschnitt durch das Dentalimplantat gemäß Schnittlinie II-II nach Fig. 1,
Fig. 3
   einen Querschnitt des Dentalimplantats gemäß Schnittlinie III-III nach Fig. 1,
Fig. 4
   eine Seitenansicht des Dentalimplantats gemäß Fig. 1 als Normalgewinde-Implantat,
Fig. 5
   eine Seitenansicht eines Schmalgewinde-Implantats,
Fig. 6
   eine Seitenansicht eines Breitgewinde-Implantats, und
Fig. 7
   eine schematische Seitenansicht eines Finalbohrers.

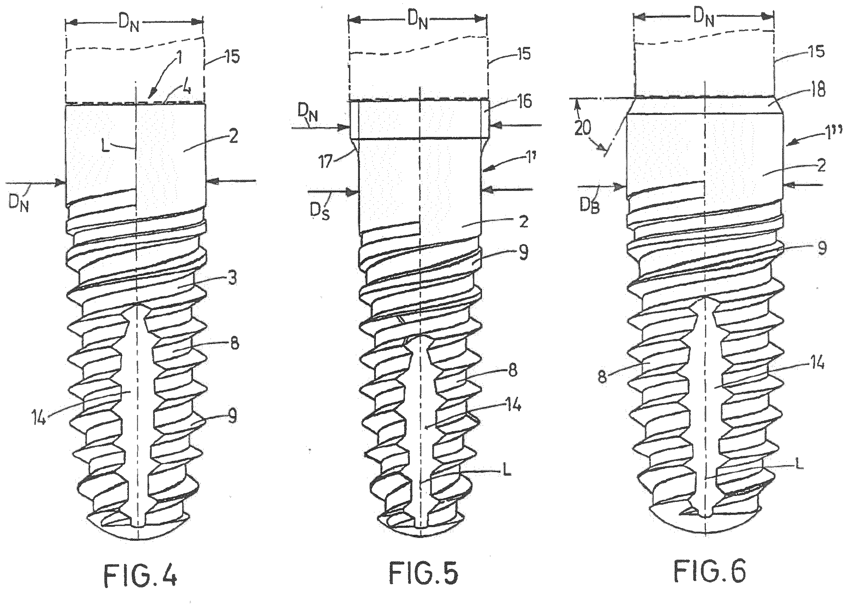

Wie aus Fig. 1, 3 und 4 deutlich wird, weist ein Dentalimplantat einen in seiner Außenkontur streng zylindrischen Verankerungskopf 2 und einen sich daran in apikaler Richtung einstückig anschließenden Schraubkörper 3 auf. Der Verankerungskopf 2 weist in seiner crestal weisenden Verankerungsfläche 4 koaxial mit der Längsachse L eine Innensechsrund-artige Innenverzahnung 5 auf, die als Formschlusselement für ein Implantierungswerkzeug bzw. als Drehsicherungselement für ein darin einzusteckendes und mit einer separaten Schraube zu verankerndes Aufbauteils (Fig. 4 bis 6) dient. An die Innenverzahnung 5 schließt sich in apikaler Richtung eine Sackbohrung 6 mit einem Innengewinde 7 an, mit dem diese Verankerungsschraube zur Festlegung des Aufbauteils 6 verschraubbar ist. Während sich die Innenverzahnung 5 im Wesentlichen über die Länge des Verankerungskopfes 2 erstreckt, nimmt die Sackbohrung 6 etwa ein Drittel der Länge des Schraubkörpers 3 ein.

Wie insbesondere aus Fig. 1 hervorgeht, weist der Schraubkörper 3 einen Gewindekern 8 und ein selbstschneidendes Außengewinde 9 auf. In Richtung der Längsachse L weisen dabei Gewindekern 8 und Außengewinde 9 drei crestal nach apikal aufeinander folgende Abschnitte auf, von denen der erste der sich an den Verankerungskopf 2 anschließende Crestalabschnitt 10 ist. In diesem Abschnitt weist das Außengewinde 9 einen konstanten Außendurchmesser D₉ von z. B. 4,5 mm auf, der dem Außendurchmesser des Verankerungskopfes 2 entspricht. Der Gewindekern 8 läuft in diesem Abschnitt in apikaler Richtung konisch zu und verjüngt sich von einem Durchmesser, der dem Durchmesser D₉ entspricht - der Gewindekern 8 geht also stufenlos in den Verankerungskopf 2 über - auf einen Durchmesser D₁₁, der beispielsweise in einer Größenordnung eines knappen halben Millimeters geringer ist als der Außendurchmesser D₉ des Verankerungskopfes 2.

An den Crestalabschnitt 10 schließt sich ein Mittenabschnitt 12 an, in dem der Außendurchmesser D₁₂ des Außengewindes 9 und der Außendurchmesser D₁₁ des Gewindekerns 8 konstant bleiben. Die scheinbare Verringerung des Außendurchmessers im Mittenabschnitt ist durch die Schnittlage in Fig. 1 durch die beiden weiter unten erwähnten Längsnuten 14 hervorgerufen.

Als dritter Abschnitt ist schließlich ein Spitzenabschnitt 13 vorgesehen, bei dem sowohl das Außengewinde 9 als auch der Gewindekern 8 in apikaler Richtung konisch auf einen Durchmesser D₁₃ bzw. D₁₄ zulaufen. Der Gewindeaußendurchmesser D₁₃ beträgt dort z. B. etwa 3,5 mm.

Zur Verdeutlichung der vorstehend erläuterten Gewindekonfiguration ist der Verlauf des Gewindekerns 8 stark strich-punktiert auf einer Seite der Schnittdarstellung gemäß Fig. 1 hervorgehoben. Auf der gleichen Seite ist ebenfalls strich-punktiert die Einhüllende E des Außengewindes 9 im Spitzenabschnitt 13 angedeutet.

Wie ferner aus den Fig. 1 und 2 hervorgeht, sind beginnend ab dem Mittenabschnitt 12 an zwei diametral gegenüber liegenden Seiten des Schraubkörpers 3 zwei parallel zur Längsachse verlaufende Längsnuten 14 eingeformt, deren Länge etwa zwei Drittel der Implantatlänge a entspricht.

Bei einem Implantat der Gesamtlänge a = 8 mm betragen die Längen L₁₀ und L₁₃ von Crestal- 10 und Spitzenabschnitt 13 lediglich 1,8 mm. Crestal- 10 und Spitzenabschnitt 13 sind also jeweils etwa gleich bis halb so lang wie der Mittenabschnitt 12.

Das Außengewinde 9 selbst weist eine Steigung von 1,8 mm auf und ist im Spitzenabschnitt 13 mit einer Anfasung versehen.

Die Fig. 4 bis 6 geben einen Satz von Dentalimplantaten 1 (Fig. 4), 1' (Fig. 5) und 1" (Fig. 6) wieder, deren Gewindekonfiguration der vorstehend beschriebenen entspricht. Insoweit bedarf es keiner nochmaligen Erörterung der Dentalimplantate 1', 1". Das Dentalimplantat 1 ist das Normalgewinde-Implantat 1 des Satzes, bei dem der Verankerungskopf 2 eine streng zylindrische Außenform mit dem Normal- Außendurchmesser D_{N} (entsprechend D₉) aufweist. Dementsprechend weist auch die Verankerungsfläche 4 diesen Normal-Außendurchmesser D_{N} auf. Das in der Innenverzahnung 5 drehfest gehaltene und über das Innengewinde 7 mit einer nicht gezeigten Schraube gesicherte Aufbauteil 15, das in den Fig. 4 bis 6 jeweils strichliert angedeutet ist, weist den gleichen Normal-Außendurchmesser D_{N} auf. Insoweit ist zwischen Aufbauteil 15 und Verankerungskopf 2 ein vollkommen glatter Übergang.

Das in Fig. 5 gezeigte Schmalgewinde-Implantat 1' weist einen Außendurchmesser D_{S} von Außengewinde 9 und Verankerungskopf 2 auf, der gegenüber dem Normal-Außendurchmesser D_{N} beispielsweise um 0,5 mm geringer ist. Damit die Verankerungsfläche 4 wieder einen Durchmesser aufweist, der dem Normal-Durchmesser D_{N} entspricht, weist der Verankerungskopf 2 am crestalen Rand eine stegartige, umlaufende Verbreiterungsschulter 16 auf, deren Außendurchmesser dem Normal-Durchmesser D_{N} entspricht. Die Stufe zwischen dem Verankerungskopf 2 und der Verbreitungsschulter 16 ist dabei mit einer ausgeprägten Innenrundung 17 versehen, sodass auch hier keine scharfen Hinterschneidungen an dem Dentalimplantat 1 ausgeprägt sind.

In Fig. 6 ist ein Breitgewinde-Implantat 1" gezeigt, bei dem der Außendurchmesser D_{B} des Außengewindes 9 am Crestalabschnitt 10 und damit des Verankerungskopfes 2 und beispielsweise 0,5 mm größer als der Normal-Außendurchmesser D_{N} ist. Um auch hier wieder eine entsprechende Anpassung des Durchmessers der Verankerungsfläche 4 an das Aufbauteil 15 zu erzielen, weist der Verankerungskopf 2 am crestalen Rand eine umlaufende Fase 18 auf, deren crestale Kante 19 den Normal-Außendurchmesser D_{N} umschreibt. Die Fase 18 bildet mit einer Radialebene einen Fasenwinkel 20 von 60°, sodass auch hier wieder ein relativ glatter Übergang zu dem Aufbauteil 15 geschaffen wird.

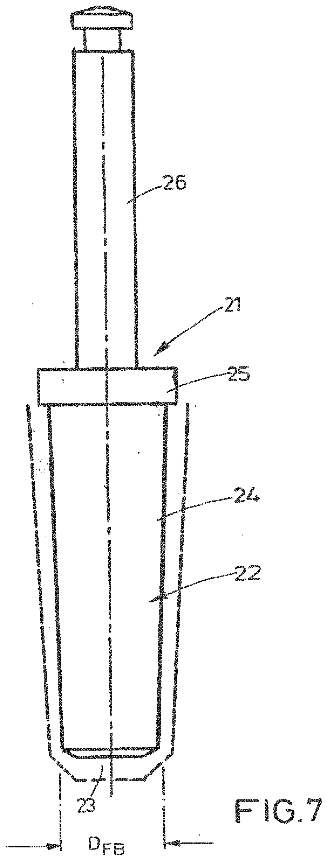

Ein Finalbohrer 21, wie er zum Anlegen einer sogenannten Finalbohrung für das in den Fig. 1 bis 4 gezeigte Dentalimplantat 1 dient, ist in Fig. 7 gezeigt. Dieser Finalbohrer 21 weist einen in seiner Außenkontur konischen Schneidkörper 22 mit drei nicht näher dargestellten Schneiden auf. Letztere erstrecken sich von der Spitze 23 ausgehend über die Mantelfläche 24 des Bohrers 1 bis zu einem Tiefenanschlag 25 in Form einer umlaufenden Ringschulter auf. Diese erstreckt sich radial über den Schneidkörper 22 hinaus und begrenzt somit die Bohrtiefe. Die Steigung der nicht dargestellten Schneiden beträgt im Übrigen beispielsweise 50 mm, sodass sich eine aktive Schneidfläche des Schneidkörpers 22 ergibt, die von der Spitze 23 bis zum Tiefenanschlag 25 reicht. Diese aktive Fläche ist in Fig. 7 durch die strichlierte, um den Schneidkörper 22 herumgeführte Linie angedeutet.

Der Schneidkörper 22 verjüngt sich zur Spitze 23 hin gleichmäßig und weist so eine konische Schneidkontur auf, deren Konuswinkel dem Konusverlauf des Gewindekerns 8 des Dentalimplantats 1 angepasst ist, d. h. etwa den dort im Crestalabschnitt 10 und Spitzenabschnitt 13 vorgesehenen Konuswinkel aufweist. Entsprechend ist auch der Außendurchmesser D_{FB} an den jeweiligen Außendurchmesser D₁₁, D₁₄ angepasst, d. h. er bewegt sich innerhalb weniger Zehntel Millimeter in der gleichen Größe.

Auf der dem Schneidkörper 22 abgewandten Seite des Tiefenanschlages 25 ist der Finalbohrer 21 mit einem Verankerungsschaft 26 versehen, mit dem der Bohrer 21 in einen entsprechenden handgeführten Antriebskopf für Dentalwerkzeuge einsetzbar ist.

## Patentansprüche

1. Dentalimplantat mit einem Verankerungskopf (2) für ein Aufbauteil (15) und einem Schraubkörper (3) mit einem Gewindekern (8) und einem selbstschneidenden Außengewinde (9), wobei der Gewindekern (8) und das Außengewinde (9) drei crestal nach apikal aufeinanderfolgende Abschnitte aufweist, nämlich einem sich an den Verankerungskopf (2) anschließenden Crestalabschnitt (10) mit konstantem Außendurchmesser (D9) des Außengewindes (9) und in apikaler Richtung konischem Gewindekern(8), einem Mittenabschnitt (12) mit weiterhin konstantem Außendurchmesser (D9) des Außengewindes (9) bei konstantem Durchmesser (D11) des Gewindekerns (8), und einem Spitzenabschnitt (13) mit in apikaler Richtung konischem Außendurchmesser (D13) des Außengewindes (9) und konischem Durchmesser (D14) des Gewindekerns (8), **dadurch gekennzeichnet, dass** das Außengewinde (9) zweigängig angelegt ist; wobei das Verhältnis der Axiallängen (L10, L12, L13) von Crestal-(10) bzw. Spitzenabschnitt (13) einerseits zum Mittenabschnitt (12) andererseits zwischen 1 : 1 und 1 : 2 liegt.

2. Dentalimplantat nach Anspruch 1, **dadurch gekennzeichnet, dass** das Verhältnis der Gewindeaußendurchmesser (D9) im Crestal- und Mittenabschnitt zu dem am apikalen Ende des Spitzenabschnittes (13) bei 4:3,5 liegt.

3. Dentalimplantat nach Anspruch 1 oder 2, **dadurch gekennzeichnet, dass** der Verankerungskopf (2) eine Innensechsrund-artige Verzahnung (5) mit sich apikal daran anschließender Gewindesackbohrung (6) aufweist.

## Claims

1. A dental implant comprising an anchoring head (2) for a mounting member (15) and a screw body (3) with a thread core (8) and a self-tapping male thread (9), wherein the thread core (8) and the male thread (9) have three portions which occur in succession in the direction crestal towards apical, namely a crestal portion (10) adjoining the anchoring head (2) and of a constant outside diameter (D9) of the male thread (9) and a thread core (8) which is conical in the apical direction, a central portion (12) with a continuing constant outside diameter (D9) of the male thread (9) with a constant outside diameter (D11) of the thread core (8), and a tip portion (13) with an outside diameter (D13) of the male thread (9), that is conical in the apical direction, and with a conical diameter (D14) of the thread core (8), **characterised in that** the male thread (9) is of a double-flight configuration, wherein the ratio of the axial lengths (L10, L12, L13) of the crestal portion (10) and the tip portion (13) respectively on the one hand to the central portion (12) on the other hand lies between 1:1 and 2: 1.

2. A dental implant according to claim 1 **characterised in that** the ratio of the thread outside diameter (D9) in the central portion relative to that at the apical end of the tip portion (13) is 4:3.5.

3. A dental implant according to claim 1 or 2 **characterised in that** the anchoring head (2) has a hexalobular-like tooth configuration (5) with a thread blind bore (6) apically adjoining same.

## Revendications

1. Implant dentaire avec une tête d'ancrage (2) pour une partie de reconstruction (15) et un corps vissé (3) avec un noyau fileté (8) et un filet mâle autotaraudeur (9), le noyau fileté (8) et le filet mâle (9) présentant trois sections successives de la crête au sommet, à savoir une section de crête (10) avec un diamètre extérieur constant (D9) du filet mâle (9) se raccordant à la tête d'ancrage (2) et un noyau fileté (8) conique en direction apicale, une section médiane (12) avec un diamètre extérieur (D9) restant constant du filet mâle (9) pour un diamètre constant (D11) du noyau fileté (8), et une section de pointe (13) avec un diamètre extérieur (D13) conique en direction apicale du filet mâle (9) et un diamètre conique (D14) du noyau fileté (8), **caractérisé en ce que** le filet mâle (9) est conçu à double pas, cependant le rapport des longueurs axiales (L10, L12, L13) d'une part de la section de crête (10) respectivement de la section de pointe (13) par rapport à, d'autre part, de la section médiane (12) se situe entre 1 : 1 et 2 : 1.

2. Implant dentaire suivant la revendication 1, **caractérisé en ce que** le rapport du diamètre extérieur du filet (D9) dans la section médiane par rapport à celui à l'extrémité apicale de la section de pointe (13) se situe vers 4 : 3,5.

3. Implant dentaire suivant la revendication 1 ou 2, **caractérisé en ce que** la tête d'ancrage (2) présente une denture à clé hexalobulaire interne (5) avec un trou aveugle taraudé (6) s'y raccordant du côté apical.
